# EUROPEAN PATENT APPLICATION

(11) **EP 4 115 903 A1**
(43) Date of publication of application: **11.01.2023**
(21) Application number: 21765093.6
(22) Date of filing: 01.03.2021
(51) Int. Cl.: A61K 39/395, A61K 47/26, A61K 47/10, C07K 16/22, A61P 35/00, A61P 35/04

(54) **PHARMACEUTICAL FORMULATION COMPRISING BEVACIZUMAB**

(30) Priority: 04.03.2020 CN 202010143839
(71) Applicant: Shanghai Henlius Biotech, Inc., Shanghai 201210 (CN)
(72) Inventor: LIU, Mujun, Shanghai 201210 (CN); FANG, Yuan, Shanghai 201210 (CN); HAN, Dongmei, Shanghai 201210 (CN)
(74) Representative: Rentsch Partner AG
(86) International application number: PCT/CN2021/078395
(87) International publication number: WO 2021/175175

(57) **Abstract**

Provided is a pharmaceutical formulation comprising 10-80 mg/ml of bevacizumab. The buffer system of the pharmaceutical formulation is preferably 10-30 mM histidine hydrochloride-sodium acetate buffer, in which 25-50 mg/mL of sucrose or sorbitol is used as a stabilizer, and Tween 80 is used as a surfactant, and the system has a pH of 5.0-5.6. The formulation is tested under repeated freezing and thawing and high-temperature acceleration conditions of 40°C. The protein stability is good, and is better than other similar formulations.

## Description

### Technical Field

The present invention belongs to the technical field of biomedicine, and in particular relates to a pharmaceutical formulation comprising bevacizumab.

### Background Art

Highly specific, effective and safe protein (antibody) drugs, especially therapeutic antibody drugs, have become a global hotspot for drug development. Bevacizumab (Chinese trade name: 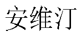 ^{®}; English trade name: Avastin^{®}) is developed by Roche, and is first approved for marketing by the US FDA in 2004 for widely used in the treatment of a variety of malignant tumors, such as metastatic colorectal cancer, non-small cell lung cancer, renal cell cancer, ovarian cancer, cervical cancer and glioblastoma. Bevacizumab is a humanized monoclonal IgG1 antibody, and can specifically bind to the vascular endothelial growth factor, thus blocking its binding to receptors (Flt-1 and KDR) on the surface of endothelial cells, avoiding a series of subsequent cascade reactions, inhibiting the neogenesis of abnormal blood vessels, thereby preventing the growth and spread of tumors, and finally achieving the purpose of eliminating tumors. Furthermore, bevacizumab has a strong specificity, and usually does not interfere with other targets while blocking the VEGF pathway. Since bevacizumab can destroy abnormal blood vessels and normalize mature blood vessels, it is usually used for combined chemotherapy, that is, used together with other drugs against tumor tissues. In such therapies, bevacizumab can effectively assist and consolidate the therapeutic effect of other drugs (Presta L G, Chen H, O\'Connor S J, Chisholm V, Meng YG, Krummen L, et al. Cancer Res 1997;57:4593-9).

In the study of monoclonal antibody drugs, the study of pharmaceutical formulations plays an important role. IgG1 monoclonal antibodies are mainly used as liquid formulations for injection, and the protein in the liquid formulations is apt to form aggregates or particles, which will affect the stability. Maintaining the good physical, chemical and biological stability of monoclonal antibody liquid formulations during the storage has become an issue that cannot be ignored. There is an urgent need for the development of stable protein formulations which can meet the requirements of the pharmaceutical industry.

### Summary of the Invention

It is an objective of the present invention to provide a pharmaceutical formulation comprising bevacizumab.

According to the present invention, the buffer system is examined with bevacizumab (HLX04 protein) as the subject of the study by means of screening and formula optimization; and the effects of different ion strengths, pH, stabilizer type, types and content of the surfactant, etc. in the buffer system on the protein stability are examined under high-temperature acceleration condition by designing a single-factor experiment. The proportion range of the content of each component in the formulation is experimentally determined.

According to the present invention, the detection items involved in the evaluation of the stability of the protein in the formulation under high-temperature acceleration condition include: appearance, protein concentration (A280), osmolality, purity (SEC-HPLC, CEX-HPLC, and CE-SDS), average particle size of the protein, PdI (DLS), and the number of sub-visible particles (FlowCam).

A preferred pharmaceutical formulation of the present invention includes: bevacizumab, a buffer, a stabilizer, and a surfactant, wherein the bevacizumab is a recombinant humanized monoclonal antibody with a content of preferably 10-100 mg/mL, preferably 10-80 mg/mL, or 10-50 mg/mL, and more preferably 10 mg/mL, 25 mg/mL, 50 mg/mL, and 80 mg/mL.

Preferably, the buffer of the present invention includes: one of a histidine-histidine hydrochloride system, an acetic acid-sodium acetate system, and a histidine hydrochloride-sodium acetate system; more preferably, the buffer is an acetic acid-sodium acetate buffer system, or a histidine hydrochloride-sodium acetate buffer system; and most preferably, the buffer is a histidine hydrochloride-sodium acetate buffer system.

The pH of the pharmaceutical formulation is preferably 5.0-5.6, and preferably 5.3.

In the buffer system, the histidine-histidine hydrochloride buffer, acetic acid-sodium acetate buffer, and histidine hydrochloride-sodium acetate buffer are preferably at a concentration of 10-30 mM, wherein the histidine hydrochloride-sodium acetate buffer is preferably at a concentration of 10 mM.

The formulation of the present invention also comprises a stabilizer for maintaining the stability of the protein drug and maintaining the function of the protein drug without being affected by changes in the conditions (such as changes in freezing, lyophilization, or other preparation conditions). The stabilizer is preferably selected from: one or more of sucrose, trehalose, mannitol, sorbitol or glycine; and the stabilizer is preferably sucrose, trehalose, and sorbitol, and more preferably is sucrose and sorbitol. The content of the stabilizer is preferably: 20-100 mg/mL; more preferably: 25-50 mg/mL; and preferably: 45 mg/mL.

The surfactant is a conventional surfactant in the art, and preferably, a nonionic surfactant. An example of the surfactant herein is preferably polysorbate, and more preferably, Tween 80. The content of the surfactant is preferably 0.1-0.5 mg/mL, and preferably 0.2 mg/mL.

The dosage forms of the pharmaceutical formulation are conventional dosage forms in the art, preferably including a liquid formulation or lyophilized formulation for injection. The liquid formulation for injection preferably includes a formulation for subcutaneous injection, a formulation for intravenous injection, a formulation for intraperitoneal administration, a formulation for intramuscular injection, a formulation for intravenous/subcutaneous injection or a formulation for intravitreal injection. The liquid formulation for injection preferably includes a formulation of aqueous solution for injection and a formulation for prefilled syringe injection, preferably the formulation of aqueous solution for injection, and the formulation of aqueous solution for injection can be used for intravenous injection or intravitreal injection.

A preferred formulation of bevacizumab is determined on the basis of the single-factor study results and the composition thereof is as follows: 25 mg/mL of bevacizumab, 10 mM of histidine hydrochloride-sodium acetate (pH 5.3), 45 mg/mL of sorbitol, and 0.2 mg/mL of Tween 80. According to the above-mentioned proportion, a finished product is prepared and the stability of the formulation formula of the present invention is verified by means of accelerated stability study and repeated freezing and thawing stability study. The formulations of the present application are compared with different formulations in stability tests. It can be seen according to the analysis of the results regarding molecular isomers, charge isomers and sub-visible particles in high-temperature acceleration tests that the stability of bevacizumab in the formulation of the present invention is significantly better than that of other formulations.

### Brief Description of the Drawings

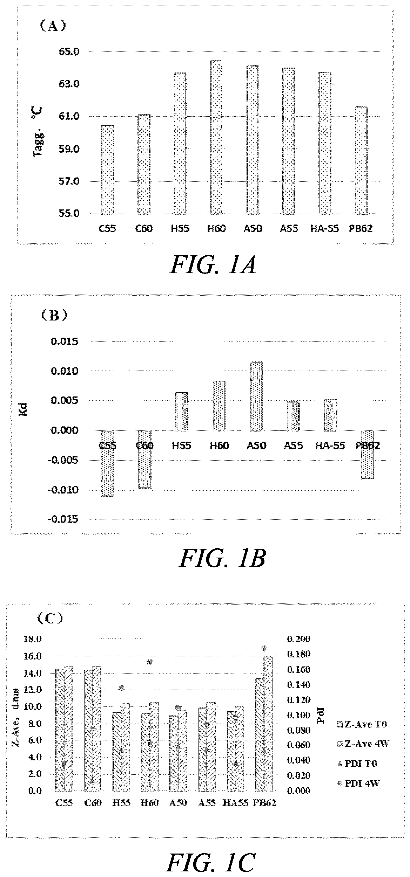

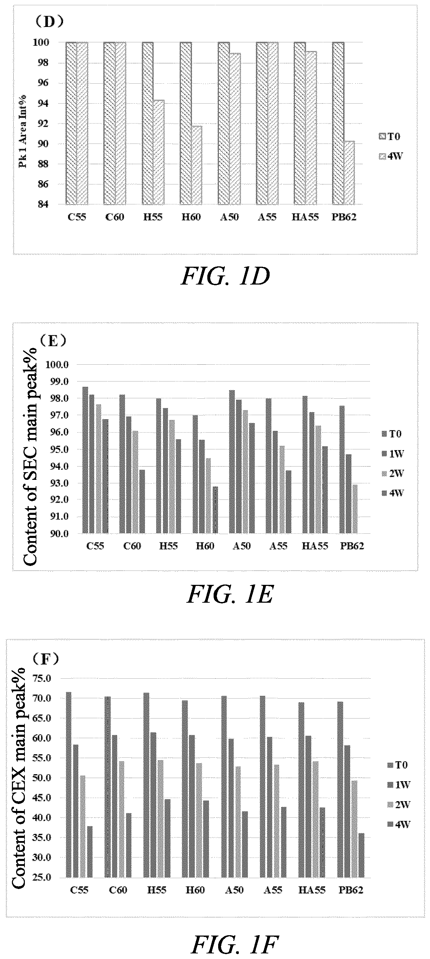

Figure 1 Examination of the physicochemical properties of the HLX04 protein in the buffer systems of the formulations shown in Table 5,
   wherein (A) illustrates the Tagg temperatures at week 0, (B) illustrates the KD values at week 0; (C) illustrates the average particle sizes and polydispersity indexes (PDI) of the HLX04 protein in the buffer systems at week 0 and after standing at 40°C for 4 weeks; (D) illustrates the Pk 1 Area percentage of the HLX04 protein in the buffer system at week 0 and after standing at 40°C for 4 weeks; (E) change trend of the content of SEC aggregates at 40°C; and (F) change trend of the content of CEX main peaks at 40°C.

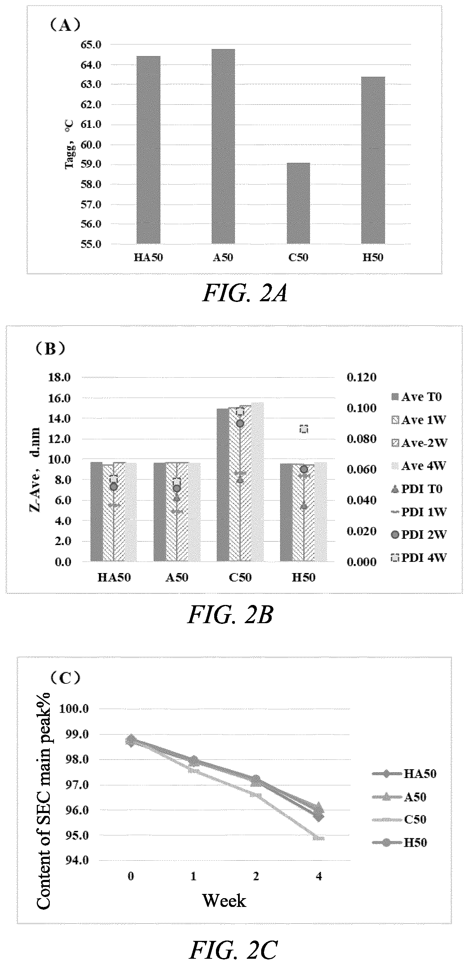

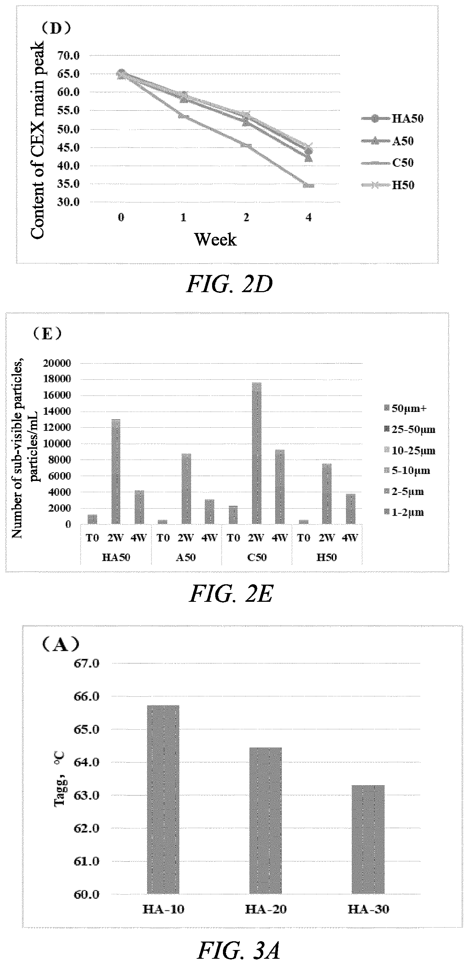

Figure 2 Examination of the physicochemical properties of the HLX04 protein in the buffer systems of the formulations shown in Table 8,
   wherein (A) is a diagram indicating thermodynamic stability comparison, (B) shows change trend of the average particle size of the protein at 40°C; (C) shows change trend of the content of SEC aggregates at 40°C; (D) shows change trend of the content of CEX main peaks at 40°C; and (E) shows change trend of the number of sub-visible particles (by FlowCam) at 40°C.

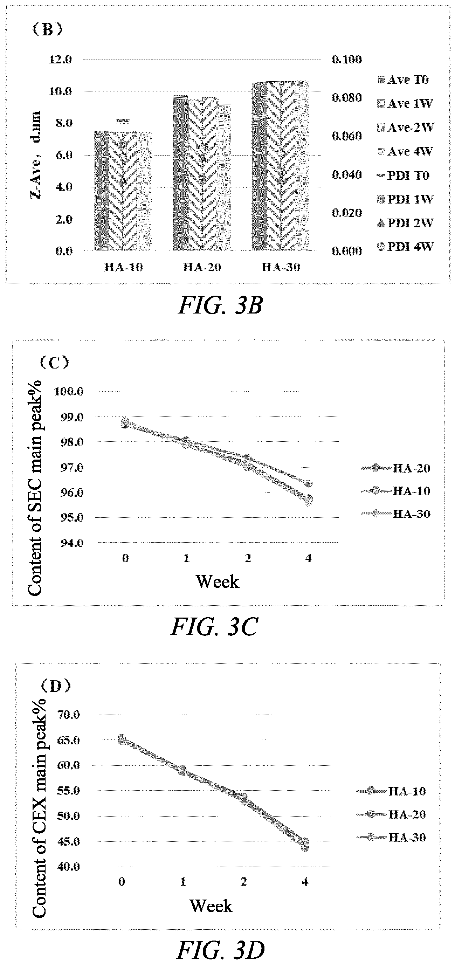

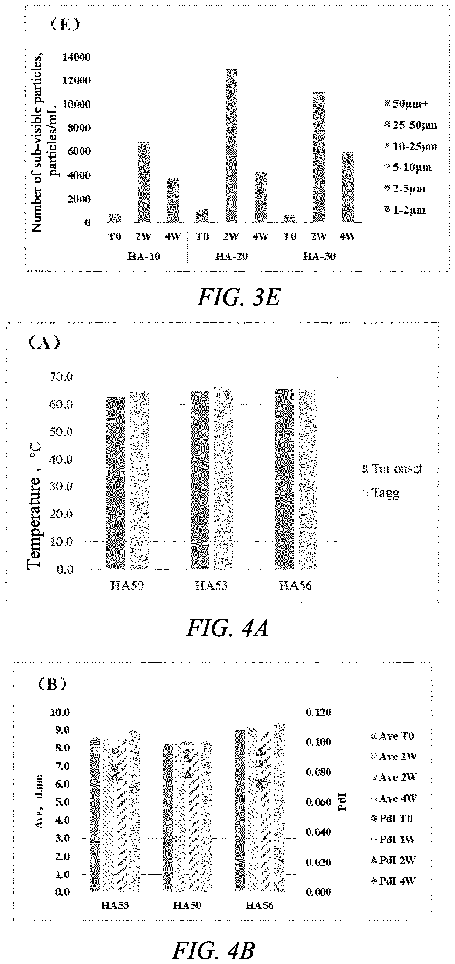

Figure 3 Examination of the physicochemical properties of the HLX04 protein in the buffer systems of the formulations shown in Table 11,
   wherein (A) is a diagram indicating thermodynamic stability comparison, (B) shows change trend of the average particle size of the protein at 40°C; (C) shows change trend of the content of SEC aggregates at 40°C; (D) shows change trend of the content of CEX main peaks at 40°C; and (E) shows change trend of the number of sub-visible particles (by FlowCam) at 40°C.

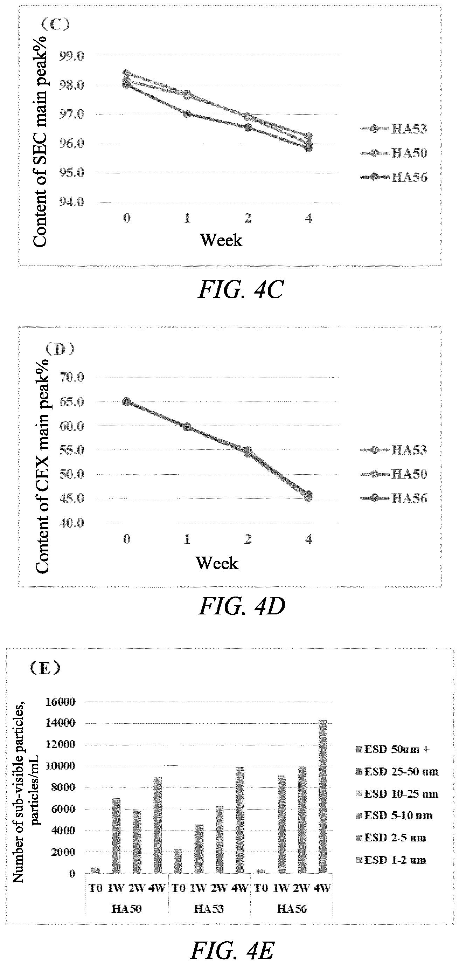

Figure 4 Examination of the physicochemical properties of the HLX04 protein in the buffer systems of the formulations shown in Table 14,
   wherein figure (A) shows a diagram indicating thermodynamic stability comparison, (B) shows change trend of the average hydrodynamic size and PdI at 40°C measured by DLS; (C) shows change trend of the content of SEC aggregates at 40°C; (D) shows change trend of the content of CEX main peaks at 40°C; and (E) shows change trend of the number of sub-visible particles (by FlowCam) at 40°C.

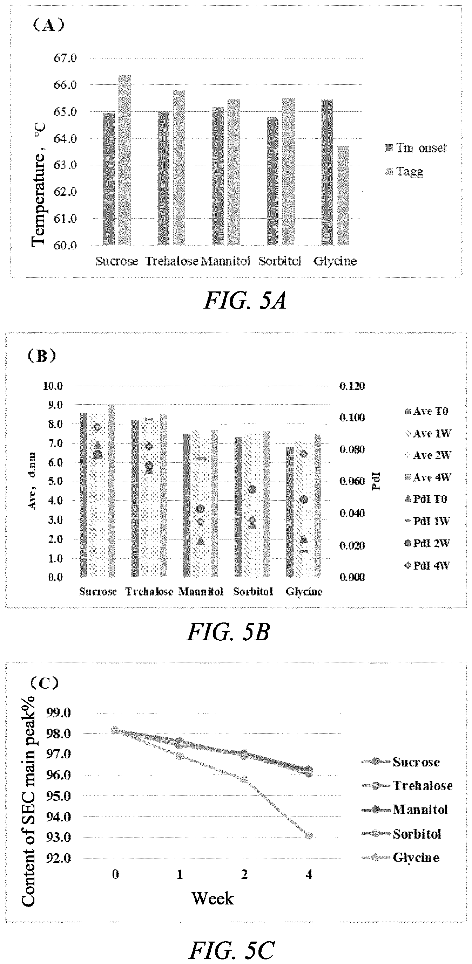

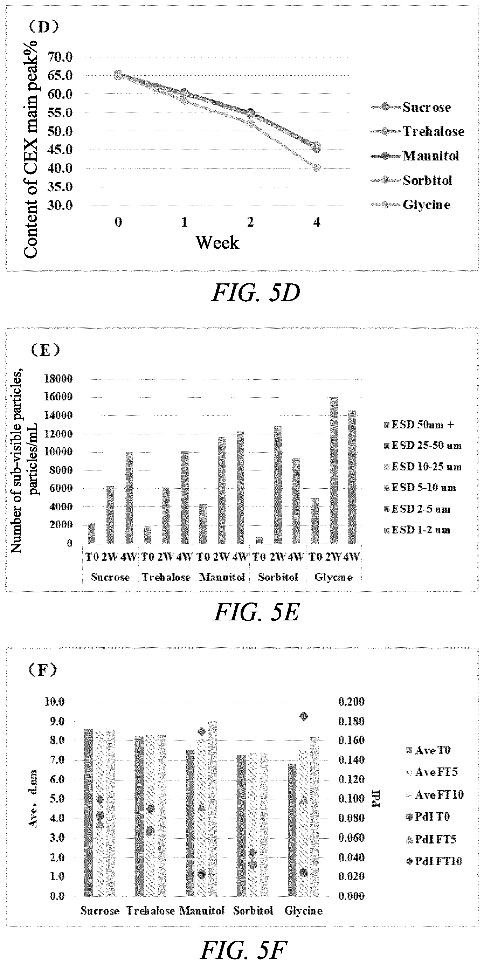

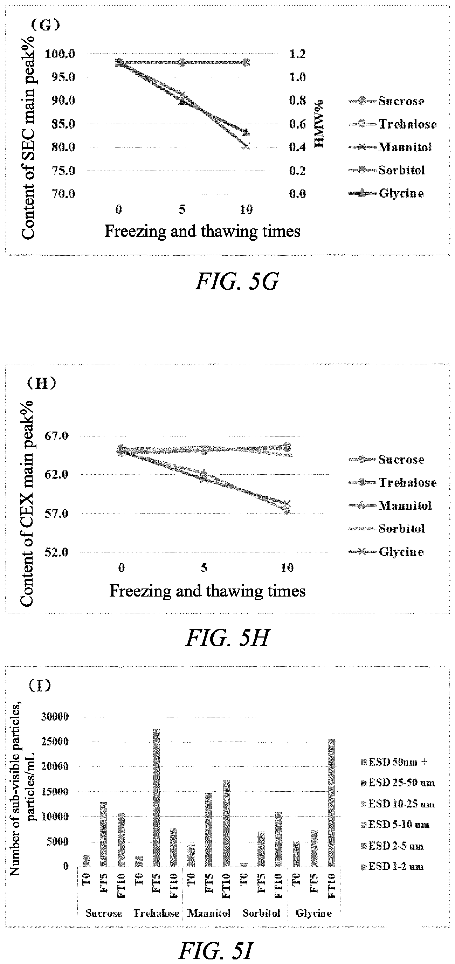

Figure 5 Examination of the physicochemical properties of the HLX04 protein in the buffer systems of the formulations shown in Table 20, wherein (A) shows a diagram indicating thermodynamic stability comparison; (B) shows change trend of average hydrodynamic size and PdI measured by DLS at 40°C; (C) shows change trend of the content of SEC aggregates at 40°C; (D) shows change trend of the content of CEX main peaks at 40°C; and (E) shows change trend of sub-visible particles (by FlowCam) at 40°C; (F) shows change trend of average hydrodynamic size and PdI measured by DLS at -20°C to room temperature; (G) shows change trend of the content of SEC aggregates at -20°C to room temperature; (H) shows change trend of the content of CEX main peaks at -20°C to room temperature; and (I) shows change trend of the number of sub-visible particles (by FlowCam) at -20°C to room temperature.

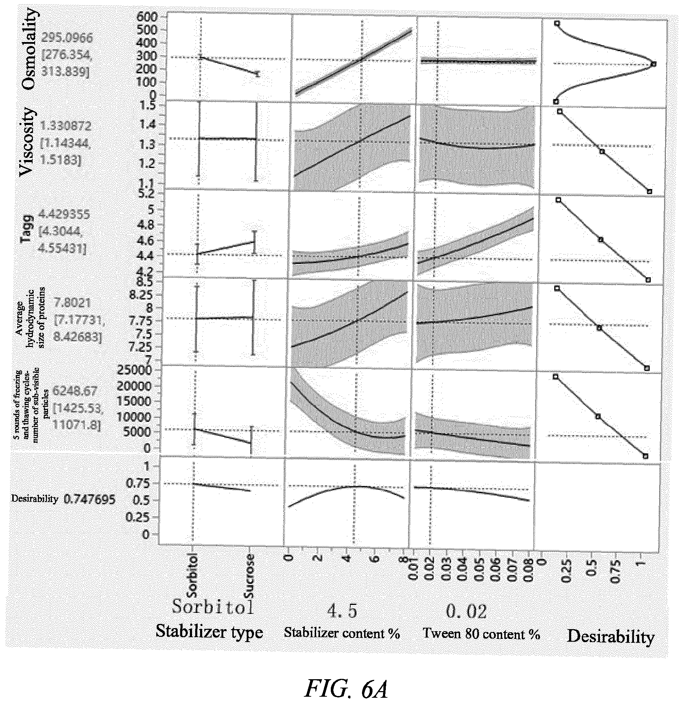

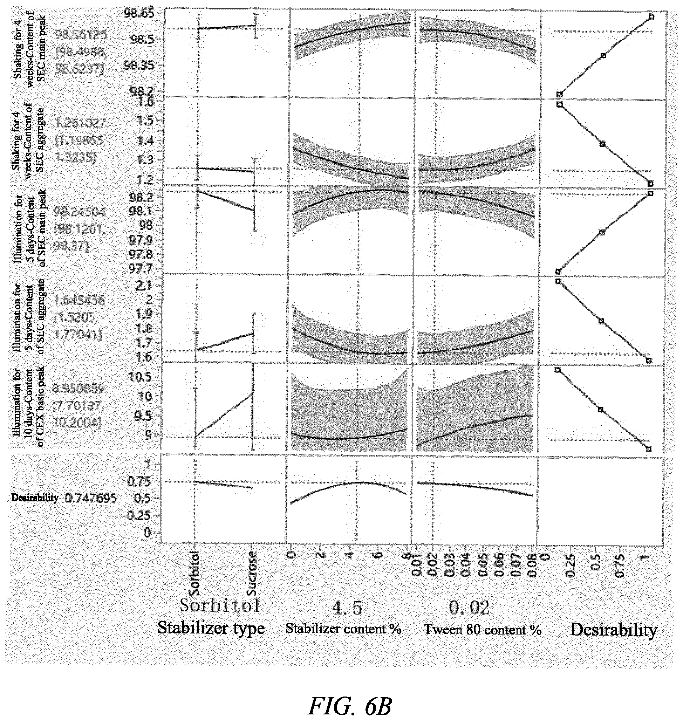

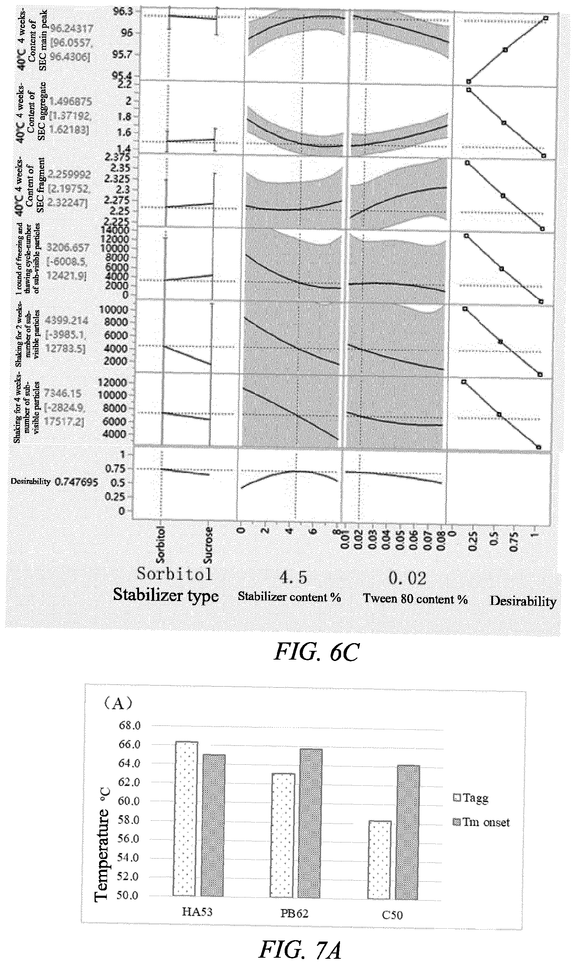

Figures 6 (A), (B) and (C) The desirability profiler model diagrams obtained by using the JMP software.

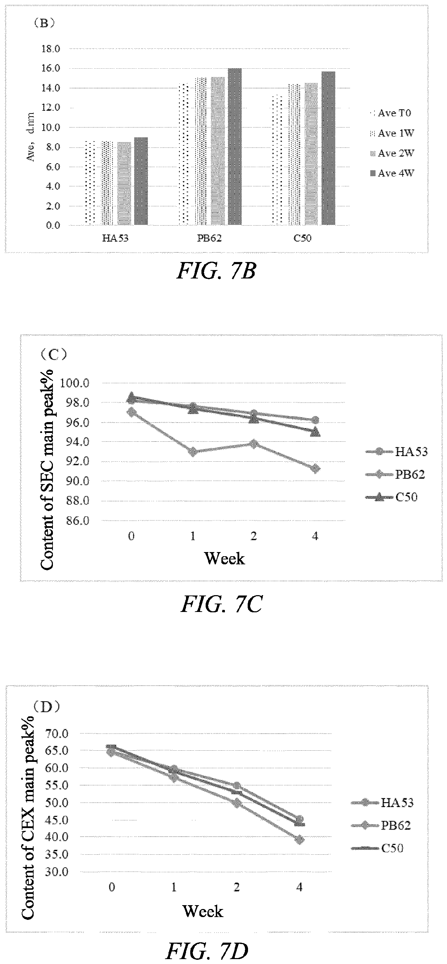

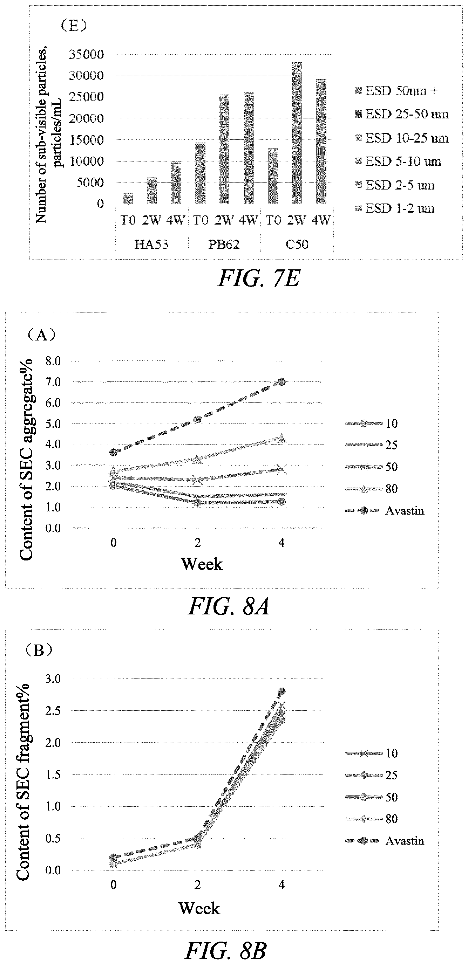

Figure 7 Examination of the physicochemical properties of the HLX04 protein in the buffer systems of the formulations shown in Table 26, (A) shows a diagram indicating thermodynamic stability comparison; (B) shows change trend of the average particle size of the protein at 40°C measured by DLS; (C) shows change trend of the content of SEC main peaks at 40°C; (D) shows change trend of the content of CEX main peaks at 40°C; and (E) shows change trend of the number of sub-visible particles (by FlowCam) at 40°C.

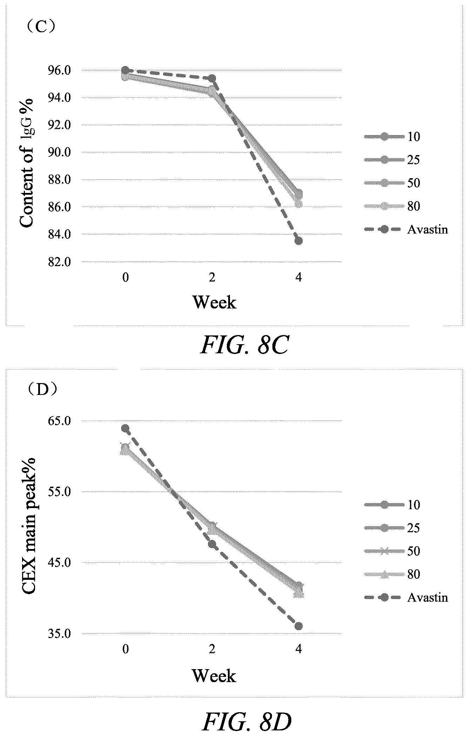

Figure 8 Comparison diagrams of the stability at different protein concentrations, (A) show change trend of the content of SEC aggregates; (B) shows change trend of the content of SEC fragments; (C) shows change trend of the content of IgG (CE-SDS non-reducing); and (D) shows change trend of the content of CEX main peaks.

### Detailed Description of Embodiments

The following examples are intended to provide those of ordinary skill in the art with a complete disclosure and description regarding how to implement and use the present invention, and are not intended to limit the scope of the invention, nor are they intended to mean that the experiments below are all the experiments implemented and the only experiments that can be implemented.

The chemical reagents used in the examples are all commercially available analytical reagents, and the recombinant monoclonal antibody can be a monoclonal antibody prepared according to any known method. The following exemplary antibody preparation method is provided by Shanghai Henlius Biotechnology Co., Ltd., and the exemplary method does not limit the present invention.

The antibody protein used in this study is HLX04 (bevacizumab), and the antibody is prepared according to the conventional method in the prior art, wherein the light and heavy chain sequences thereof are as follows:
Light chain 
DIQMTQSPSSLSASVGDRVTITCSASQDISNYLNWYQQKPGKAPKVLIYFTSSLHSGVPS
RFSGSGSGTDFTLTISSLQPEDFATYYCQQYSTVPWTFGQGTKVEIKRTVAAPSVFIFPP
SDEQLKSGTASVVCLLNNFYPREAKVQWKVDNALQSGNSQESVTEQDSKDSTYSLSSTLT
LSKADYEKHKVYACEVTHQGLSSPVTKSFNRGEC (SEQ ID NO:1)
Heavy chain

EVQLVESGGGLVQPGGSLRLSCAASGYTFTNYGMNWVRQAPGKGLEWVGWINTYTGEPT
YAADFKRRFTFSLDTSKSTAYLQMNSLRAEDTAVYYCAKYPHYYGSSHWYFDVWGQGTL
VTVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPS
REEMTKNQVSLTCLVKGFYPSDIAVEWESNGQPENNYKTTPPVLDSDGSFFLYSKLTVDK
SRWQQGNVFSCSVMHEALHNHYTQKSLSLSPGK (SEQ ID NO:2)

### Example 1. Detection method

### 1.1. Appearance and visible particles

The appearance is detected by visual inspection. The sample bottle is wiped clean and placed in SC-4000A clarity tester (Huanghai Medicine & Drug Testing Instruments, Shanghai) in a dark room, the illumination intensity is adjusted to 1000-1500 Lux, the sample is placed at the edge (25 cm) of the light blocking plate, and the color, clarity, visible particles, etc. are visually inspected against a black ground and a white background, respectively while holding the neck of the sample penicillin bottle.

### 1.2. Protein content

The absorbance of the sample at the wavelength of 280 nm is measured and the protein concentration is calculated by using a Trinean Dropsense16 protein concentration meter. The extinction coefficient is 1.60 mL^{∗}mg^{-1∗}cm⁻¹.

### 1.3. pH

The sample (25 µL) is taken and the pH thereof is determined by using a Mettler Toledo multifunctional parameter meter, which is calibrated with three standard solutions (the pH being 4.01, 7.00 and 9.21, respectively), so that the electrode slope is in the range of 95%-105%.

### 1.4. Osmolality

The determination is carried out according to General Rule 0632 "Determination of Osmolality", Pharmacopoeia of the People's Republic of China (2015 Edition) by using an Advanced Osmo PRO osmometer. The sample (20 µL) and two 290 mOsmol/kg of osmolality standards are taken, and the osmolality values of the sample and the standards are determined by using the cryoscopic method.

### 1.5. Viscosity

The viscosity of the sample is measured by using the BROOKFIELD DV2T viscometer. The external water bath kettle is switched on, the temperature is set at 25°C, 0.5 mL of the sample is suctioned up and dropwise added into the center of the sample cup, the rotor speed is set so that the measurement torque falls within 40%-60%, and the viscosity of the sample is determined.

### 1.6. DLS

The particle size and particle size distribution of the sample are determined by using the DynaPro PlateReader-III high-throughput dynamic and static light scattering instrument. In a clean bench, the sample (25 µL) is taken and added to the microwells of a 384-well plate, and after the addition is completed, the plate is covered with a film. The film-covered 384-well plate is placed into a refrigerated centrifuge and centrifuged to remove the bubbles in the microwells of the sample. The specific parameter settings of the instrument are shown in **Table 1.**

**Table 1 Parameter settings of high-throughput dynamic and static light scattering instrument for particle size detection**

| **Module** | **Parameter** | **Set value** |
|---|---|---|
| Experiment | Type of experiment | Isothermal |
| | Automatic attenuation | Yes |
| | Image of each sample well | Yes |
| | Acquisition time of dynamic light scattering | 5 s |
| | Number of dynamic light scattering acquisition of each sample well | 10 |
| | Detection of static light | No |
| Duplicate detection | Detection number of each sample well | 1 |
| | Waiting time between scannings for sample wells | 0 min |
| | Number of scannings | 1 |
| | Waiting time between scannings | 0 min |
| Temperature | Onset temperature | 25°C |
| | Sample plate | Sealing |
| End of the experiment | Temperature | 25°C |
| | Detector | Off |

### 1.7. Tagg temperature

The aggregation temperature of the sample is determined by using the DynaPro PlateReader-III high-throughput dynamic and static light scattering instrument. In a clean bench, the sample (25 µL) is taken and added to the microwells of a 384-well plate, and after the addition is completed, the plate is covered with a film. The film-covered 384-well plate is placed into a refrigerated centrifuge and centrifuged to remove the bubbles in the microwells of the sample. The specific parameter settings of the instrument are shown in Table 2.

**Table 2 Parameter settings of high-throughput dynamic and static light scattering instrument for Tagg detection**

| **Module** | **Parameter** | **Set value** |
|---|---|---|
| Experiment | Type of experiment | Thermal Ramp |
| | Automatic attenuation | Yes |
| | Image of each well | Yes |
| | Acquisition time of dynamic light scattering | 5 s |
| | Number of dynamic light scattering acquisition of each sample well | 5 |
| | Detection of static light | No |
| Temperature | Onset temperature | 40°C |
| | End temperature | 80°C |
| | Sample plate | Sealing |
| End of the experiment | Temperature | 25°C |
| | Detector | Off |

### 1.8. DSC

The thermodynamic parameters T_{m onset}, Tₘ₁ and Tₘ₂ values related to the protein conformation are determined by using the TA Nano DSC differential scanning calorimeter. The protein sample is diluted to a concentration of 1 mg/mL with a placebo, and the diluted protein sample and the corresponding placebo are respectively placed in a 96-well sample plate, and after degassing treatment, placed under a pressure condition of 300 ± 50 Kpa. The pre-equilibration time is set as 600 s, the temperature range is set as 25°C-100°C, and the scanning rate is set as 1°C/min. The DSC curves of the protein sample and placebo are collected, respectively, with three times of scanning for the placebo and one scanning for the protein sample. The Two State Scaled model is selected for data fitting.

### 1.9. SEC-HPLC

The SEC-HPLC detection is carried out by using the Agilent 1260 high-performance liquid chromatography with a TOSOH TSKgel G3000 chromatographic column (7.8 mm × 300 mm, 5 µm). The column temperature is room temperature (without controlling the temperature), the temperature of the sample tray is 2°C-8°C, the mobile phase consists of 100 mM of sodium dihydrogen phosphate and 0.5% of sodium chloride, the pH is 6.8, and isocratic elution is carried out. The elution time is 30 min, and the flow rate is 0.5 mL/min. The detection wavelength is 280 nm, the concentration of the sample is diluted to 1 mg/mL, and the injection volume is 50 µL.

### 1.10. CEX-HPLC

The CEX-HPLC detection is carried out by using the Agilent 1260 high-performance liquid chromatography with a Thermo ProPac^{™}WCX-10 chromatographic column (4 mm × 250 mm, 10 µm). The column temperature is 35°C, the sample tray temperature is 2°C-8°C, the mobile phase A consists of 50 mM of phosphate buffer (the pH being 6.10), the mobile phase B (the pH being 6.10) consists of 50 mM of phosphate buffer and 300 mM of sodium chloride, and gradient elution is carried out. The elution gradient can be seen in Table 3, and the flow rate is 1.0 mL/min. The detection wavelength is 280 nm, the concentration of the sample is diluted to 1 mg/mL, and the injection volume is 50 µL.

**Table 3 Elution gradient of CEX-HPLC**

| **Retention time** | **Mobile phase B%** | **Flow rate (mL/min)** |
|---|---|---|
| 0.0 min | 4.0% | 1.0 |
| 3.0 min | 4.0% | 1.0 |
| 31.0 min | 31.0% | 1.0 |
| 32.0 min | 100.0% | 1.0 |
| 34.0 min | 100.0% | 1.0 |
| 34.1 min | 4.0% | 1.0 |
| 39.0 min | 4.0% | 1.0 |

### 1.11. CE-SDS

The determination is carried out according to General Rule 3127 "Determination of Molecular Size Heterogeneities in Monoclonal Antibodies (CE-SDS method)", Pharmacopoeia of the People's Republic of China (2015 Edition). Detection is carried out using non-reducing and reducing CE-SDS. The Beckman Coulter PA800 plus capillary electrophoresis apparatus and an uncoated capillary with a total length of 67 cm and an inner diameter of 50 µm are used. Injection: 5 KV, 20.0 s; separation: 15 KV, 35.0 min, and detection is carried out at the wavelength of 220 nm by using a PDA detector; and calculation is carried out by the area normalization method.

### 1.12. FlowCam

The morphology and number of sub-visible particles in the sample are determined by using the FlowCam 8100 particle analytical detector. The specific parameter settings of the instrument are shown in Table 4.

**Table 4 Detection parameter settings of FlowCam 8100 particle analytical detector**

| **Category** | **Parameter** | **Set value** |
|---|---|---|
| Capture | Distance to Nearest Neighbor (µm) | 0 |
| | Close Holes | 2 |
| Segmentation Threshold | Dark Pixels | 15 |
| | Light Pixels | 15 |
| | Both Dark and Light Pixels | Selected |
| Flow cell | Flow cell type | FC80FV |
| | Flow cell depth (µm) | 81 |
| | Flow cell width (µm) | 700 |
| Fluidics | Sample volume, mL | 0.4 |
| | Flow rate, mL/min | 0.1 |
| | Efficiency% | About 70% |

### Example 2 Buffer system and pH screening study

For the study of buffer system/pH, two rounds of experiments are carried out, and the type and pH range of the buffer system are screened by designing a single-factor experiment.

### 2.1 Buffer system/pH screening study-I

### 2.1.1 Study protocol

**Table 5 Formulation information of buffer system/pH screening study-I**

| **No.** | **Buffer type** | **Ionic strength, mmol/L** | **pH** | **Code** |
|---|---|---|---|---|
| B1 | Citrate-sodium citrate | 20 | 5.5 | C55 |
| B2 | | | 6.0 | C60 |
| B3 | Histidine-histidine hydrochloride | 20 | 5.5 | H55 |
| B4 | | | 6.0 | H60 |
| B5 | Acetic acid-sodium acetate | 20 | 5.0 | A50 |
| B6 | | | 5.5 | A55 |
| B7 | Histidine hydrochloride-sodium acetate | 20 | 5.5 | HA55 |
| B8 | Sodium dihydrogen phosphate-disodium hydrogen phosphate | 50 | 6.2 | PB62 |

In this study, the HLX04 protein stock solution (batch number: AS201801) in which Tween 20 has been removed by using cation chromatography is subjected to ultrafiltration and medium exchange, and then the protein concentration is adjusted to prepare an alternative formulation with a final protein concentration of about 25.0 mg/mL (Table 5). In a biosafety cabinet, the sample is filtered by using a 0.22 µm disposable sterile filter and then 1 mL of the protein solution is aseptically sub-packaged into 2 mL penicillin vials. The vials are capped with 13 mm rubber stoppers, and 13 mm aluminum-plastic composite caps are used to seal the stoppers. The sub-packaged samples are placed in a constant temperature and humidity chamber at 40°C for storage, and are sampled and detected according to the design requirements in Table 6.

**Table 6 Examination conditions and detection methods of buffer system/pH screening study-I**

| **Examination conditions** | **Week 0** | **Week 1** | **Week 2** | **Week 4** |
|---|---|---|---|---|
| 40°C | X, Y, Z | X | X | X, Y |
| X: Appearance, protein content (A₂₈₀), SEC, and CEX | | | | |
| Y: pH, DLS | | | | |
| Z: Tagg, KD | | | | |

### 2.1.1 Study results

At week 0, the Tagg temperature is relatively higher (Figure 1A), the KD value is positive (Figure 1B), and the average particle size is relatively smaller (Figure 1C) for the protein in the histidine-histidine hydrochloride, acetic acid-sodium acetate and histidine hydrochloride-sodium acetate systems, indicating that the protein shows better conformational and colloidal stability in the three buffer systems.

After standing at 40°C for 4 weeks, the results of protein concentrations and pH show no significant difference (Table 7). The SEC results show that the contents of the main peaks of the proteins in each buffer system all show a downward trend (Figure IE), and in all the three buffer systems of citrate-sodium citrate, histidine-histidine hydrochloride and acetic acid-sodium acetate, the lower is the pH, the better is the stability. The formulations are ranked from superiority to inferiority as C55>A50>H55>HA55>C60≈A55>H60 (Figure 1E). The DLS results show that the polydispersity indexes (PdI) of the protein increase (Figure 1C), and the percentage of Pk 1 Area Int decrease (Figure 1D) in the buffer systems of histidine-histidine hydrochloride and sodium dihydrogen phosphate-disodium hydrogen phosphate, indicating that a soluble high polymer of less than 1 µm is produced. The CEX results show that the contents of the main peaks of the proteins in each buffer system all show a downward trend (Figure IF), and the stability of the protein in the histidine-histidine hydrochloride buffer system is significantly better than that in the other buffer systems.

**Table 7 Data summary of buffer system/pH screening study-I**

| **Detection item** | **Examinati on Time** | **B1** | **B2** | **B3** | **B4** | **B5** | **B6** | **B7** | **B8** |
|---|---|---|---|---|---|---|---|---|---|
| | | **C55** | **C60** | **H55** | **H60** | **A50** | **A55** | **HA55** | **PB62** |
| Protein concentration (mg/mL) | Week 0 | 25.4 | 25.1 | 25.0 | 24.8 | 25.3 | 25.6 | 25.1 | 25.2 |
| | Week 1 | 25.3 | 25.4 | 25.4 | 25.1 | 25.3 | 25.6 | 25.0 | 24.9 |
| | Week 2 | 25.4 | 25.4 | 24.8 | 25.1 | 25.3 | 25.1 | 25.0 | 24.8 |
| | Week 4 | 25.3 | 25.3 | 25.3 | 25.1 | 25.3 | 25.5 | 25.2 | 25.0 |
| pH | Week 0 | 5.6 | 6.0 | 5.5 | 6.0 | 5.2 | 5.7 | 5.6 | 6.2 |
| | Week 4 | 5.5 | 6.0 | 5.6 | 6.0 | 5.1 | 5.7 | 5.6 | 6.2 |

### 2.2 Buffer system/pH screening study-II

### 2.2.1 Study protocol

The results of the buffer system/pH screening study-I show that in the histidine-histidine hydrochloride, acetic acid-sodium acetate and citrate-sodium citrate buffer systems, the lower is the pH, the higher is the content of the SEC main peak; and with regard to the proteins in the formulations without stabilizers and surfactants, after being placed at 40°C examination conditions for 1 week, there are particles visible to the naked eye in each of the formulations. Therefore, in this round of experiment, the buffer system screening is further performed in the formulation with sucrose and Tween 80 at a low pH of 5.0.

**Table 8 Formulation information of buffer system/pH screening study-II**

| **No.** | **Buffer type** | **Ionic strength, mmol/L** | **pH** | **Stabilizer** | **Surfactant** | **Code** |
|---|---|---|---|---|---|---|
| B1 | Histidine hydrochloride-sodium acetate | 20 | 5.0 | 50 mg/mL of sucrose | 0.2 mg/mL of Tween 80 | HA50 |
| B2 | Acetic acid-sodium acetate | | | | | A50 |
| B3 | Citrate-sodium citrate | | | | | C50 |
| B4 | Histidine-histidine hydrochloride | | | | | H50 |

In this study, the HLX04 protein (batch number: AS201901-PT) is subjected to ultrafiltration and medium exchange, then auxiliary materials are added, and the protein concentration is adjusted to prepare an alternative formulation with a final protein concentration of about 25.0 mg/mL (Table 8). In a biosafety cabinet, the sample is filtered by using a 0.22 µm disposable sterile filter and then 1 mL of the protein solution is aseptically sub-packaged into 2 mL penicillin vials. The vials are capped with 13 mm rubber stoppers, and 13 mm aluminum-plastic composite caps are used to seal the stoppers. The sub-packaged samples are placed in a constant temperature and humidity chamber at 40°C for storage, and are sampled and detected according to the design requirements in Table 9.

**Table 9 Examination conditions and detection methods of buffer system/pH screening study-II**

| **Examination conditions** | **Week 0** | **Week 1** | **Week 2** | **Week 4** |
|---|---|---|---|---|
| 40°C | X, Y, Z | X | X, Y | X, Y |
| X: Appearance, protein content (A₂₈₀), pH, SEC, CEX, and DLS | | | | |
| Y: FlowCam | | | | |
| Z: Osmolality, Tagg | | | | |

### 2.2.2 Study results

At week 0, there is no significant difference in the basic physicochemical detection results of the proteins in each buffer system (Table 10), and the formulations are ranked from superiority to inferiority according to the Tagg aggregation temperatures are A50≈HA50>H50>C50 (Figure 2A) and are ranked from superiority to inferiority according to the average particle size of the protein as A50≈HA50≈H50>C50 (Figure 2B). It indicates that the protein shows better conformational and colloidal stability in the acetic acid-sodium acetate and histidine hydrochloride-sodium acetate buffer systems.

After standing at 40°C for 4 weeks, there is no significant difference in the basic physicochemical detection results (Table 10). The SEC results show that the contents of the main peaks of the proteins in each buffer system all show a downward trend, and the formulations are ranked from superiority to inferiority as A50≈H50≈HA50>C50 (Figure 2C). The CEX results show that the contents of the main peaks of the proteins in each buffer system all show a downward trend, and the formulations are ranked from superiority to inferiority as H50≈HA50>A50>C50 (Figure 2D). The formulations are ranked from superiority to inferiority according to the FlowCam sub-visible particles as A50>H50≈HA50>C50 (Figure 2E).

**Table 10 Data summary of buffer system/pH screening study-II**

| | | | | | |
|---|---|---|---|---|---|
| **Detection item** | **Examination time** | **B1** | **B2** | **B3** | **B4** |
| | | **HA50** | **A50** | **C50** | **H50** |
| Protein concentration (mg/mL) | Week 0 | 24.9 | 25.0 | 25.5 | 24.7 |
| | Week 1 | 25.1 | 25.3 | 26.0 | 25.3 |
| | Week 2 | 24.4 | 24.7 | 25.3 | 24.5 |
| | Week 4 | 24.3 | 24.6 | 25.3 | 24.5 |
| pH | Week 0 | 5.1 | 5.1 | 5.1 | 5.0 |
| | Week 1 | 5.0 | 5.1 | 5.1 | 5.0 |
| | Week 2 | 5.1 | 5.1 | 5.1 | 5.1 |

| **Detection item** | **Examination time** | **B1** | **B2** | **B3** | **B4** |
|---|---|---|---|---|---|
| | | **HA50** | **A50** | **C50** | **H50** |
| | Week 4 | 5.1 | 5.1 | 5.1 | 5.0 |
| Osmolality (mOsmol/kg) | Week 0 | 202 | 214 | 219 | 203 |

### 2.2.3 Study conclusion

The results of this round of study show that the protein shows better conformational and colloidal stability in the acetic acid-sodium acetate and histidine hydrochloride-sodium acetate buffer systems. The results regarding charge isomers of the protein in the histidine-histidine hydrochloride buffer system are better, and after comprehensive consideration, histidine hydrochloride-sodium acetate is selected as the buffer system of the protein.

### Example 3 Screening of ionic strength

### 3.1 Study protocol

**Table 11 Formulation information of ionic strength screening study**

| **No.** | **Buffer type** | **Ionic strength, mmol/L** | **pH** | **Stabilizer** | **Surfactant** | **Code** |
|---|---|---|---|---|---|---|
| B1 | Histidine hydrochloride-sodium acetate | 10 | 5.0 | 50 mg/mL of sucrose | 0.2 mg/mL of Tween 80 | HA-10 |
| B2 | | 20 | | | | HA-20 |
| B3 | | 30 | | | | HA-30 |

In this study, the HLX04 protein (batch number: AS201901-PT) is subjected to ultrafiltration and medium exchange, then auxiliary materials are added, and the protein concentration is adjusted to prepare an alternative formulation with a final protein concentration of about 25.0 mg/mL (Table 11). In a biosafety cabinet, the sample is filtered by using a 0.22 µm disposable sterile filter and then 1 mL of the protein solution is aseptically sub-packaged into 2 mL penicillin vials. The vials are capped with 13 mm rubber stoppers, and 13 mm aluminum-plastic composite caps are used to seal the stoppers. The sub-packaged samples are placed in a constant temperature and humidity chamber at 40°C for storage, and are sampled and detected according to the design requirements in Table 12.

**Table 12 Examination conditions and detection methods of ionic strength screening study**

| **Examination conditions** | **Week 0** | **Week 1** | **Week2** | **Week 4** |
|---|---|---|---|---|
| 40°C | X, Y, Z | X | X, Y | X, Y |
| X: Appearance, protein content (A₂₈₀), pH, SEC, CEX, and DLS | | | | |
| Y: FlowCam | | | | |
| Z: Osmolality, Tagg | | | | |

### 3.2 Study results

At week 0, the formulations are ranked from superiority to inferiority according to the Tagg aggregation temperatures (Figure 3A) and average particle sizes (Figure 3B) of the proteins in the histidine hydrochloride-sodium acetate systems as HA-10>HA-20>HA-30, indicating that the protein shows better conformational and colloidal stability in a 10 mM of histidine hydrochloride-sodium acetate buffer system.

After standing at 40°C for 4 weeks, there is no significant difference in the basic physicochemical detection results (Table 13). The SEC results show that the contents of the main peaks of the proteins in each buffer system all show a downward trend (Figure 3C), and the formulations are ranked from superiority to inferiority as HA-10>HA-20≈HA-30. The CEX results show that the contents of the main peaks of the proteins in each buffer system all show a downward trend, and there is no significant difference in the downward trends (Figure 3D). The formulations are ranked from superiority to inferiority according to the FlowCam sub-visible particles as HA-10>HA-20≈HA-30 (Figure 3E).

**Table 13 Data summary of ionic strength screening study**

| **Detection item** | **Examination time** | **B1** | **B2** | **B3** |
|---|---|---|---|---|
| | | **HA-10** | **HA-20** | **HA-30** |
| Protein concentration (mg/mL) | Week 0 | 25.1 | 24.9 | 24.8 |
| | Week 1 | 25.3 | 25.1 | 25.3 |
| | Week 2 | 24.5 | 24.4 | 24.5 |
| | Week 4 | 24.5 | 24.3 | 24.5 |
| pH | Week 0 | 5.1 | 5.1 | 5.0 |
| | Week 1 | 5.1 | 5.0 | 5.0 |

| **Detection item** | **Examination time** | **B1** | **B2** | **B3** |
|---|---|---|---|---|
| | | **HA-10** | **HA-20** | **HA-30** |
| | Week 2 | 5.2 | 5.1 | 5.1 |
| | Week 4 | 5.2 | 5.1 | 5.1 |
| Osmolality (mOsmol/kg) | Week 0 | 189 | 202 | 225 |

### 3.3 Study conclusion

The results of this round of study show that the protein shows better conformational and colloidal stability in 10 mM of histidine hydrochloride-sodium acetate buffer system. The content of the SEC main peak shows a slower downward trend. There are few FlowCam sub-visible particles.

### Example 4 Screening of pH range

### 4.1 Study protocol

**Table 14 Formulation information of pH range screening study**

| **No.** | **Buffer type** | **Ionic strength, mmol/L** | **pH** | **Stabilizer** | **Surfactant** | **Code** |
|---|---|---|---|---|---|---|
| B1 | Histidine hydrochloride-sodium acetate | 10 | 5.0 | 100 mg/mL of sucrose | 0.2 mg/mL of Tween 80 | HA50 |
| B2 | | | 5.3 | | | HA53 |
| B3 | | | 5.6 | | | HA56 |

In this study, the HLX04 protein (batch number: AS201901-PT) is subjected to ultrafiltration and medium exchange, then auxiliary materials are added, and the protein concentration is adjusted to prepare an alternative formulation with a final protein concentration of about 25.0 mg/mL (Table 14). In a biosafety cabinet, the sample is filtered by using a 0.22 µm disposable sterile filter and then 1 mL of the protein solution is aseptically sub-packaged into 2 mL penicillin vials. The vials are capped with 13 mm rubber stoppers, and 13 mm aluminum-plastic composite caps are used to seal the stoppers. The sub-packaged samples are placed in a constant temperature and humidity chamber at 40°C for storage, and are sampled and detected according to the design requirements in Table 15.

**Table 15 Examination conditions and detection methods of pH range screening study**

| **Examination conditions** | **Week 0** | **Week 1** | **Week 2** | **Week 4** |
|---|---|---|---|---|
| 40°C | X, Y | X | X | X |
| X: Appearance, protein content (A₂₈₀), pH, SEC, CEX, DLS, and FlowCam | | | | |
| Y: DSC, Tagg | | | | |

### 4.2 Study results

The proteins are stored in 10 mM of histidine hydrochloride-sodium acetate system for 4 weeks, and there is no significant difference in the physicochemical properties of the proteins in the buffers in the pH range of 5.0-5.6 (Table 16, Figure 4). Therefore, the pH range of the final formulation is 5.0-5.6.

**Table 16 Data summary of pH range screening study**

| **Detection item** | **Examination time** | **B1** | **B2** | **B3** |
|---|---|---|---|---|
| | | **HA50** | **HA53** | **HA56** |
| Protein concentration (mg/mL) | Week 0 | 25.0 | 25.2 | 25.5 |
| | Week 1 | 25.4 | 25.6 | 25.5 |
| | Week 2 | 25.8 | 25.9 | 25.8 |
| | Week 4 | 26.3 | 25.9 | 26.0 |
| pH | Week 0 | 5.0 | 5.4 | 5.6 |
| | Week 1 | 5.0 | 5.4 | 5.6 |
| | Week 2 | 5.0 | 5.4 | 5.6 |
| | Week 4 | 5.0 | 5.4 | 5.6 |

### Example 5 Screening of stabilizer type

### 5.1 Study protocol

**Table 17 Formulation information of stabilizer type screening study**

| **No.** | **Buffer type** | **Ionic strength, mmoI/L** | **pH** | **Stabilizer** | **Surfactant** | **Code** |
|---|---|---|---|---|---|---|
| F1 | Histidine hydrochlor | 10 | 5.3 | 100 mg/mL of sucrose | 0.2 mg/mL of Tween 80 | Sucrose |

| **No.** | **Buffer type** | **Ionic strength, mmol/L** | **pH** | **Stabilizer** | **Surfactant** | **Code** |
|---|---|---|---|---|---|---|
| F2 | ide-sodium acetate | | | 100 mg/mL of trehalose | | Trehalose |
| F3 | | | | 50 mg/mL of mannitol | | Mannitol |
| F4 | | | | 50 mg/mL of sorbitol | | Sorbitol |
| F5 | | | | 25 mg/mL of glycine | | Glycine |

In this study, the HLX04 protein (batch number: AS201901-PT) is subjected to ultrafiltration and medium exchange, then auxiliary materials are added, and the protein concentration is adjusted to prepare an alternative formulation with a final protein concentration of about 25.0 mg/mL (Table 17). In a biosafety cabinet, the sample is filtered by using a 0.22 µm disposable sterile filter and then 1 mL of the protein solution is aseptically sub-packaged into 2 mL penicillin vials. The vials are capped with 13 mm rubber stoppers, and 13 mm aluminum-plastic composite caps are used to seal the stoppers. The sub-packaged samples are examined and detected according to the design requirements in Table 18.

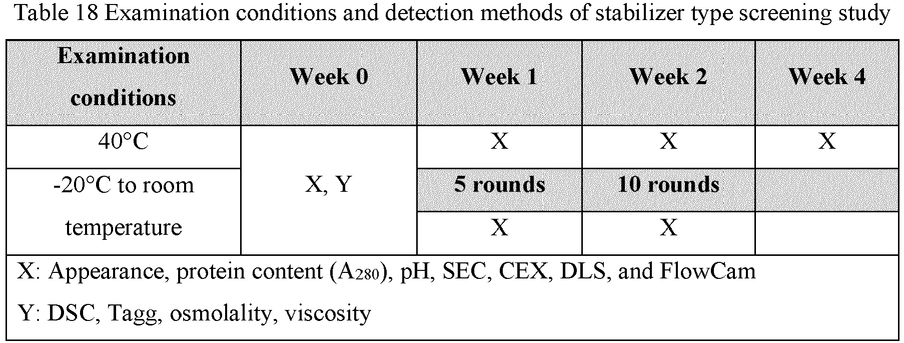

### 5.2 Study results

At week 0, the stabilizers are ranked from superiority to inferiority according to the T_{agg} temperatures of the proteins in the formulations containing various stabilizers as sucrose>trehalose>sorbitol≈mannitol>glycine; and the stabilizers are ranked from superiority to inferiority according to the T_{m onset} as glycine>mannitol≈sucrose≈trehalose≈sorbitol (Figure 5A).

After standing at 40°C for 4 weeks, the SEC results show that the contents of the main peaks of the proteins in the formulations containing various stabilizers all show a downward trend, and the samples containing glycine show a significantly faster downward trend (Figure 5C). The stabilizers are ranked from superiority to inferiority according to the DLS results as sorbitol≈mannitol>glycine>sucrose≈trehalose (Figure 5B). The CEX results show that the contents of the main peaks of the proteins in each of the formulations containing various stabilizers all show a downward trend, and the samples containing glycine show a significantly faster downward trend (Figure 5D). FlowCam results show that the stabilizers are ranked from the superiority to inferiority according to the sub-visible particles as sorbitol≈sucrose≈trehalose≈mannitol>glycine (Figure 5E).

After 10 rounds of repeated freezing and thawing cycles, the SEC results show that there is no change in the contents of the main peaks of the proteins in the formulations containing sucrose, trehalose and sorbitol compared with those at week 0, and the contents of the main peaks in the formulations containing mannitol or glycine show a significantly faster downward trend (Figure 5G). The stabilizers are ranked from superiority to inferiority according to the DLS results as sorbitol>sucrose≈trehalose>glycine≈mannitol (Figure 5F). The CEX results show that there is no change in the contents of the main peaks of the proteins in the formulations containing sucrose, trehalose and sorbitol compared with those at week 0, and the contents of the main peaks in the formulations containing mannitol and glycine show a significantly faster downward trend (Figure 5H). FlowCam results show that the stabilizers are ranked from the superiority to inferiority according to the sub-visible particles as sorbitol≈sucrose≈trehalose>mannitol≈glycine (Figure 51 and Table 19).

The results of this round of study show that under the conditions of standing at the high temperature of 40°C and repeated freezing and thawing, in the formulation containing a stabilizer sucrose or sorbitol in 10 mM of histidine hydrochloride-sodium acetate buffer system (pH 5.3), the protein shows better stability.

**Table 19 Data summary of stabilizer type screening study**

| **Examination conditions** | **Detection item** | **Examination time** | **F1** | **F2** | **F3** | **F4** | **F5** |
|---|---|---|---|---|---|---|---|
| | | | **Sur** | **Tre** | **Man** | **Sor** | **Gly** |
| 40°C | Protein concentration (mg/mL) | Week 0 | 25.2 | 25.2 | 24.3 | 24.4 | 24.6 |
| | | Week 1 | 25.6 | 25.2 | 24.5 | 24.6 | 24.6 |
| | | Week 2 | 25.9 | 25.5 | 24.7 | 24.9 | 24.8 |
| | | Week 4 | 25.9 | 25.6 | 25.0 | 25.3 | 25.0 |
| | pH | Week 0 | 5.4 | 5.3 | 5.3 | 5.3 | 5.4 |
| | | Week 1 | 5.4 | 5.3 | 5.4 | 5.4 | 5.4 |
| | | Week 2 | 5.4 | 5.4 | 5.4 | 5.3 | 5.5 |
| | | Week 4 | 5.4 | 5.4 | 5.4 | 5.5 | 5.6 |
| -20°C to room temperature | Protein concentration (mg/mL) | Week 0 | 25.2 | 25.2 | 24.3 | 24.4 | 24.6 |
| | | 5 rounds | 25.2 | 24.8 | 24.3 | 24.5 | 24.4 |
| | | 10 rounds | 25.5 | 25.1 | 24.7 | 24.5 | 24.5 |
| | pH | Week 0 | 5.4 | 5.3 | 5.3 | 5.3 | 5.4 |
| | | 5 rounds | 5.4 | 5.3 | 5.3 | 5.4 | 5.5 |
| | | 10 rounds | 5.3 | 5.3 | 5.3 | 5.3 | 5.4 |
| Osmolality ( mOsmol/kg) | | Week 0 | 372 | 345 | 336 | 321 | 363 |
| Viscosity (cP) | | Week 0 | 1.6 | 1.6 | 1.4 | 1.4 | 1.2 |

### Example 6 Screening of stabilizer and surfactant

### 6.1 Study protocol

In this round of study, three factors, namely stabilizer type, stabilizer content and Tween 80 content, are selected using the JMP 15 software, 10 experimental groups (Table 20) are designed by using the Box-Behnken response surface method, and the concentrations of the stabilizer and surfactant are determined by means of accelerated conditions such as freezing and thawing, shaking, illumination and high temperature (Table 21).

**Table 20 Formulation information of stabilizer and surfactant screening study**

| **No.** | **Buffer system** | **Stabilizer type** | **Stabilizer content, mg/mL** | **Tween 80 content, mg/mL** |
|---|---|---|---|---|
| F1 | 10 mM Histidine hydrochloride-sodium acetate pH 5.3 | Sorbitol | 0 | 0.1 |
| F2 | | | 0 | 0.8 |
| F3 | | | 40 | 0.45 |
| F4 | | | 40 | 0.45 |
| F5 | | | 80 | 0.1 |
| F6 | | | 80 | 0.8 |
| F7 | | Sucrose | 0 | 0.45 |
| F8 | | | 40 | 0.1 |
| F9 | | | 40 | 0.8 |
| F10 | | | 80 | 0.45 |

In this study, the HLX04 protein (batch number: AS201901-PT) is subjected to ultrafiltration and medium exchange, then auxiliary materials are added, and the protein concentration is adjusted to prepare an alternative formulation with a final protein concentration of about 25.0 mg/mL (Table 20). In a biosafety cabinet, the sample is filtered by using a 0.22 µm disposable sterile filter and then 1 mL of the protein solution is aseptically sub-packaged into 2 mL penicillin vials. The vials are capped with 13 mm rubber stoppers, and 13 mm aluminum-plastic composite caps are used to seal the stoppers. The sub-packaged samples are examined and detected according to the design requirements in Table 21.

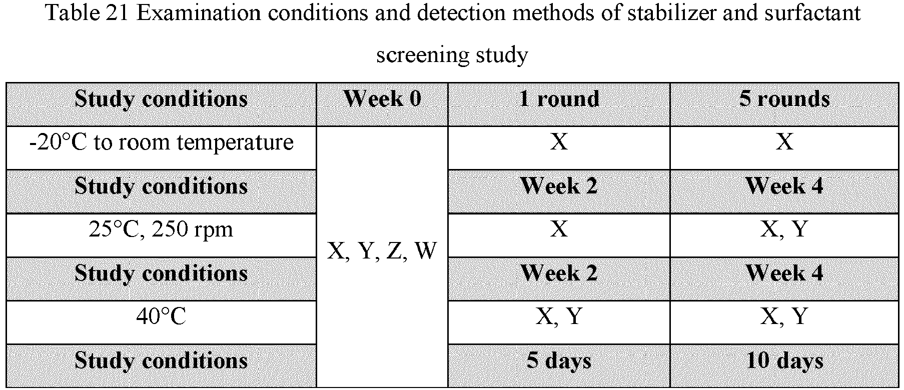

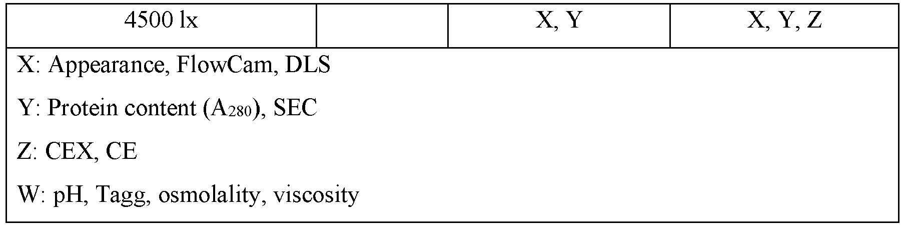

### 6.2 Study results

The detection data is imported into the JMP 15 software, and each factor (dependent variable) is subjected to multiple linear regression and binomial equation fitting by using the least square method to obtain a statistically significant (P value < 0.1) model (Table 22) with corrected coefficients of determination (R2) of greater than 0.95, indicating that the model and the actual condition are well fitted, the equation has good accuracy and reliability in terms of the response values, and the regression model can be used instead of real test points for the analysis and prediction of the experimental results.

Utilizing the JMP 15 software, the optimum formulation is predicted by using a maximized desirability model according to the analysis results in Table 22. When sorbitol is selected as the stabilizer, the desirability for the formulation is relatively higher; when the content of sorbitol is 4.5%, the desirability for the formulation is the highest; and as the content of polysorbate 80 increases, the desirability for the formulation shows a downward trend, and when the content of polysorbate 80 is in the range of 0.01%-0.03%, the desirability for the formulation is relatively higher and the midpoint of 0.02% is selected (see Figures 6A, 6B, and 6C).

According to the buffer system/pH screening study, ionic strength screening study, surfactant type screening study, stabilizer type screening study, and stabilizer and surfactant screening study, the composition of the final formulation is as follows: 10 mM of histidine hydrochloride-sodium acetate buffer system (pH 5.3), with 45 mg/mL of sorbitol and 0.2 mg/mL of Tween 80.

**Table 22 Data summary of stabilizer and surfactant screening study**

| **Examination conditions** | **Response** | **R²** | **P value** | **Factors** | | | | | | | |
|---|---|---|---|---|---|---|---|---|---|---|---|
| | | | | **Stabilizer type** | **Stabilizer content** | **Tween 80 content** | **Stabilizer type * stabilizer content** | **Stabilizer content * stabilizer content** | **Stabilizer type * Tween 80 content** | **Stabilizer content * Tween 80 content** | **Tween 80 content * Tween 80 content** |
| Week 0 | Osmolality | 0.99 | 0.0083 | 0.0079 | 0.003 | 0.2597 | 0.0104 | 0.0998 | 0.2597 | 0.6082 | 0.289 |
| | Viscosity | 0.99 | 0.1113 | 0.1875 | 0.0362 | 0.3275 | 0.6196 | 0.7332 | 0.1969 | 0.448 | 0.2508 |
| | Tagg | 0.99 | 0.033 | 0.1784 | 0.0169 | 0.0188 | 0.02 | 0.1027 | 0.0362 | 0.0321 | 0.1784 |
| | Average hydrodynamic size of proteins | 0.99 | 0.1095 | 0.1462 | 0.0371 | 0.3498 | 0.1529 | 0.2785 | 0.1282 | 0.1114 | 0.4788 |
| Freezing and thawing study | 1 round-number of sub-visible particles^{#} | 0.96 | 0.0369 | 0.051 | 0.0069 | 0.5427 | 0.057 | 0.1161 | | 0.0613 | |
| | 5 rounds-number of sub-visible particles | 0.99 | 0.0583 | 0.1056 | 0.0258 | 0.2988 | 0.0743 | 0.0368 | 0.0825 | 0.2184 | 0.7177 |
| Shaking study | 2 weeks-number of sub-visible particles^{#} | 0.97 | 0.0201 | 0.9802 | 0.0126 | 0.8786 | | 0.2381 | 0.0095 | 0.0112 | |
| | 4 weeks-number of sub-visible particles^{#} | 0.98 | 0.0163 | 0.0497 | 0.0036 | 0.1329 | 0.241 | | 0.0171 | 0.0342 | |
| | 4 weeks-content of SEC main peak | 0.99 | 0.0493 | 0.0877 | 0.0202 | 0.0362 | 0.1692 | 0.1238 | 0.2143 | 0.0473 | 0.0877 |
| | 4 weeks-content of SEC aggregate | 0.99 | 0.0496 | 0.0985 | 0.0211 | 0.0354 | 0.1103 | 0.1462 | 0.3498 | 0.0449 | 0.0846 |
| Illumination study | 5 days-content of SEC main peak | 0.99 | 0.0658 | 0.1924 | 0.0269 | 0.1529 | 1 | 0.079 | 0.0519 | 0.0499 | 0.1924 |
| | 5 days-content of SEC aggregate | 0.99 | 0.068 | 0.2785 | 0.0273 | 0.1529 | 0.7532 | 0.0846 | 0.0556 | 0.0528 | 0.2279 |
| | 10 days-content of CEX basic peak^{#} | 0.98 | 0.0619 | 0.0355 | 0.0726 | 0.4535 | 0.0332 | 0.2361 | 0.0332 | 0.0722 | |
| 40°C | 4 weeks-content of SEC main peak | 0.99 | 0.0724 | 0.1492 | 0.0406 | 0.0472 | 0.0911 | 0.0665 | 0.0987 | 0.0925 | 0.409 |
| | 4 weeks-content of SEC aggregate | 0.99 | 0.0547 | 0.1177 | 0.026 | 0.041 | 0.0967 | 0.0495 | 0.0776 | 0.0894 | 0.1663 |
| | 4 weeks-content of SEC fragment | 0.99 | 0.1347 | 0.2785 | 0.1892 | 0.0648 | 0.0967 | 0.1924 | 0.1892 | 0.0894 | 0.1924 |
| Note: Marking with # means results obtained after step-by-step analysis | | | | | | | | | | | |

### Example 7 Comparison of selected formulations with other formulations

### 7.1 Study protocol

**Table 23 Formulation information**

| **No.** | **Buffer system** | **pH** | **Stabilizer type** | **Stabilizer content, mg/mL** | **Salt** | **Surfactant type** | **Surfactant content, mg/mL** | **Code** |
|---|---|---|---|---|---|---|---|---|
| F1 | 10 mM of histidine hydrochloride-sodium acetate | 5.3 | Sorbitol | 45 | - | Tween 80 | 0.2 | HA53 |
| F2 | 50 mM of sodium dihydrogen phosphate-disodium hydrogen phosphate | 6.2 | Trehalose | 60 | - | Tween 20 | 0.4 | PB62 |
| F3 | 10 mM of citrate-sodium citrate | 5.0 | Sucrose | 20 | 100 mM of arginine hydrochloride | Tween 20 | 0.5 | C50 |

In this study, the HLX04 protein (batch number: AS201901-PT) is subjected to ultrafiltration and medium exchange, then auxiliary materials are added, and the protein concentration is adjusted to prepare an alternative formulation with a final protein concentration of about 25.0 mg/mL (Table 23). In a biosafety cabinet, the sample is filtered by using a 0.22 µm disposable sterile filter and then 1 mL of the protein solution is aseptically sub-packaged into 2 mL penicillin vials. The vials are capped with 13 mm rubber stoppers, and 13 mm aluminum-plastic composite caps are used to seal the stoppers. The sub-packaged samples are placed in a constant temperature and humidity chamber at 40°C for storage, and are sampled and detected according to the design requirements in Table 24.

**Table 24 Examination conditions and detection methods**

| **Examination conditions** | **Week 0** | **Week 1** | **Week 2** | **Week 4** |
|---|---|---|---|---|
| 40°C | X, Y, Z | X | X, Y | X, Y |
| X: Appearance, protein content (A₂₈₀), pH, SEC, CEX, DLS, and FlowCam | | | | |
| Y: DSC, Tagg, Tg' | | | | |

### 7.2 Study results

At week 0, the proteins in the 3 formulations are all colorless and slightly opalescent liquids with no obvious visible particles. The formulations are ranked from superiority to inferiority according to the Tagg aggregation temperatures as HA53>PB62>C50 (Figure 7A), and are ranked from superiority to inferiority according to the average particle sizes of the proteins as HA53>C50>PB62 (Figure 7B), indicating that the protein has better conformational and colloidal stability in the HA53 formulation.

After standing at 40°C for 4 weeks, the protein stability can be maintained to a certain degree in the 3 formulations, and there is no significant change in the basic physicochemical detections of the proteins (Table 25). The SEC results show that the contents of the main peaks of the proteins in each formulation all show a downward trend (Figure 7C), and the formulations are ranked from superiority to inferiority as HA53>C50>PB62. The CEX results show that the contents of the main peaks of the proteins in each formulation all show a downward trend (Figure 7D), and the buffer systems are ranked from superiority to inferiority as HA53>C50>PB62. The buffer systems are ranked from superiority to inferiority according to the FlowCam sub-visible particles as HA53>PB62>C50 (Figure 7E).

**Table 25 Data summary of comparative study of various formulations**

| **Detection item** | **Examination time** | **F1** | **F2** | **F3** |
|---|---|---|---|---|
| | | **HA53** | **PB62** | **C50** |
| Protein concentration (mg/mL) | Week 0 | 25.2 | 24.8 | 24.3 |
| | Week 1 | 25.6 | 25.2 | 24.5 |
| | Week 2 | 25.9 | 25.2 | 24.4 |
| | Week 4 | 25.9 | 25.2 | 24.6 |
| pH | Week 0 | 5.4 | 6.2 | 5.0 |
| | Week 1 | 5.4 | 6.1 | 5.1 |
| | Week 2 | 5.4 | 6.2 | 5.0 |
| | Week 4 | 5.4 | 6.2 | 5.1 |

According to the results regarding molecular isomers, charge isomers and sub-visible particles in high-temperature acceleration tests, the stability of bevacizumab in the formulation of the present application is significantly better than that of bevacizumab in the existing formulation (PB62) and other similar formulations.

### Example 8 Comparison of the stability at different protein concentrations

### 8.1 Study protocol

**Table 26 Formulation information**

| **No.** | **Buffer system** | **pH** | **Stabilizer type** | **Stabilizer content, mg/mL** | **Surfactant type** | **Surfactant content, mg/mL** | **Code** |
|---|---|---|---|---|---|---|---|
| F1 | 10 mM of histidine hydrochloride-sodium acetate | 5.3 | Sorbitol | 45 | Tween 80 | 0.2 | 10 |
| F2 | | | | | | | 25 |
| F3 | | | | | | | 50 |
| F4 | | | | | | | 80 |
| F5 | 50 mM of sodium dihydrogen phosphate-disodium hydrogen phosphate | 6.2 | Trehalose | 60 | Tween 20 | 0.4 | A vastin |

After the formulation screening study, it is determined that the formulation of the HLX04 formulation is as follows: 10 mM of histidine hydrochloride-sodium acetate, 45 mg/mL of sorbitol and 0.2 mg/mL of Tween 80, pH 5.3. In this round of study, under the acceleration condition of the high temperature of 40°C, the stability differences between the samples of the HLX04 formulation in the concentration range of 10-80 mg/mL and Avastin^{®} are compared. The HLX04 PT protein (batch number: AS201901-PT) is subjected to ultrafiltration and medium exchange, then auxiliary materials are added, and the protein concentration is adjusted to prepare various alternative formulations (Table 26). In a biosafety cabinet, the sample is filtered by using a 0.22 µm disposable sterile filter and then 0.5 mL of the protein solution is aseptically sub-packaged into 2 mL penicillin vials. The vials are capped with 13 mm rubber stoppers, and 13 mm aluminum-plastic composite caps are used to seal the stoppers. The sub-packaged samples and original drug (batch number: H0154B14, code: Avastin) are placed in a constant temperature and humidity chamber at 40°C for storage, and are sampled and detected according to the design requirements in Table 27.

**Table 27 Examination conditions and detection methods**

| **Examination conditions** | **Week 0** | **Week 2** | **Week 4** |
|---|---|---|---|
| 40°C | X | X | X |
| X: Appearance, protein content (A₂₈₀), pH, SEC, CEX, and CE | | | |

### 8.2 Study results

In this round of study, by means of high-temperature (40°C) acceleration tests, the stability differences between the samples of the HLX04 formulation in the concentration range of 10-80 mg/mL and Avastin^{®} are compared. The results of the study (Table 28) show that at week 0, the aggregate contents of the samples of the HLX04 formulation in the concentration range of 10-80 mg/mL are 2.0%-2.7%, which are less than the aggregate content (3.6%) of Avastin. After standing at 40°C for 4 weeks, the aggregation rate (Figure 8A), degradation rate (Figures 8B and 8C) and the change trend of charge isomers (Figure 8D) of the samples of the HLX04 formulation in the concentration range of 10-80 mg/mL are significantly slower than those of Avastin.

In summary, compared with the original drug Avastin (60 mg/mL), the HLX04 formulation in the concentration range of 10-80 mg/mL has better stability.

**Table 28 Data summary of comparison of the stability at different protein concentrations**

| **Detection item** | | **Examin ation time** | **Code** | | | | |
|---|---|---|---|---|---|---|---|
| | | | **10** | **25** | **50** | **80** | **Avastin** |
| SEC | Main peak% | Week 0 | 97.9 | 97.7 | 97.5 | 97.2 | 96.2 |
| | | Week 2 | 98.4 | 98.1 | 97.3 | 96.3 | 94.3 |
| | | Week 4 | 96.2 | 95.9 | 94.8 | 93.3 | 90.1 |
| | Aggregate % | Week 0 | 2.0 | 2.2 | 2.4 | 2.7 | 3.6 |
| | | Week 2 | 1.2 | 1.5 | 2.3 | 3.3 | 5.2 |
| | | Week 4 | 1.3 | 1.6 | 2.8 | 4.3 | 7.0 |
| | Fragment % | Week 0 | 0.1 | 0.1 | 0.1 | 0.1 | 0.2 |
| | | Week 2 | 0.4 | 0.4 | 0.4 | 0.4 | 0.5 |
| | | Week 4 | 2.6 | 2.5 | 2.4 | 2.3 | 2.8 |
| CEX | Main peak% | Week 0 | 61.2 | 60.9 | 61.2 | 60.9 | 63.9 |
| | | Week 2 | 49.9 | 50.2 | 49.9 | 49.7 | 47.6 |
| | | Week 4 | 41.1 | 41.7 | 41.2 | 40.8 | 36.0 |
| | Acid peak% | Week 0 | 30.6 | 30.8 | 30.7 | 30.6 | 27.3 |
| | | Week 2 | 41.3 | 41.2 | 40.9 | 40.5 | 42.1 |
| | | Week 4 | 49.9 | 49.5 | 49.2 | 48.6 | 52.1 |
| | Basic peak% | Week 0 | 8.2 | 8.2 | 8.2 | 8.5 | 8.8 |
| | | Week 2 | 8.7 | 8.7 | 9.2 | 9.8 | 10.4 |
| | | Week 4 | 9.0 | 8.8 | 9.5 | 10.6 | 11.9 |
| CE-SDS | IgG% | Week 0 | 95.7 | 95.6 | 95.5 | 95.6 | 96.0 |
| | | Week 2 | 94.6 | 94.5 | 94.3 | 94.5 | 95.4 |
| | | Week 4 | 87 | 86.9 | 86.8 | 86.2 | 83.5 |

## Claims

1. A pharmaceutical formulation comprising bevacizumab, wherein the pharmaceutical formulation comprises: bevacizumab, a buffer, a stabilizer, and a surfactant, wherein
the buffer is selected from one or more of a histidine-histidine hydrochloride system, an acetic acid-sodium acetate system, and a histidine hydrochloride-sodium acetate system;
the stabilizer is selected from one or more of sucrose, trehalose and sorbitol; and
the surfactant is Tween 80 or Tween 20.

2. The pharmaceutical formulation according to claim 1, wherein the buffer is a histidine hydrochloride-sodium acetate buffer at a concentration of 10-30 mM.

3. The pharmaceutical formulation according to claim 2, wherein the buffer is a histidine hydrochloride-sodium acetate buffer at a concentration of 10 mM

4. The pharmaceutical formulation according to claim 1, wherein the stabilizer is sorbitol or sucrose with a content of 20-100 mg/mL.

5. The pharmaceutical formulation according to claim 1, wherein the surfactant is Tween 80 with a content of 0.1 mg/mL-0.5 mg/mL.

6. The pharmaceutical formulation according to claim 1, wherein the pH of the pharmaceutical formulation is 5.0-5.6.

7. The pharmaceutical formulation according to claim 1, wherein the protein concentration of the bevacizumab is 10-100 mg/mL, preferably 10-80 mg/mL, or the protein concentration of the bevacizumab is 10-50 mg/mL.

8. The pharmaceutical formulation according to any one of claims 1 to 7, wherein the formulation contains: 10 mM of the histidine hydrochloride-sodium acetate buffer, 45 mg/mL of sorbitol, and 0.2 mg/mL of Tween 80, with a pH of 5.3.

9. The pharmaceutical formulation according to claim 8, wherein the formulation contains 10 mg/mL, 25 mg/mL, 50 mg/mL or 80 mg/mL of bevacizumab.

10. The pharmaceutical formulation according to any one of claim 1, wherein the formulation is a liquid formulation or lyophilized formulation for injection.
